# EUROPEAN PATENT SPECIFICATION

(11) **EP 4 023 760 B1**
(45) Date of publication and mention of the grant of the patent: **15.11.2023**
(21) Application number: 21753784.4
(22) Date of filing: 07.02.2021
(51) Int. Cl.: C12P 19/02, C12P 19/14, C12P 7/18

(54) **METHOD FOR PREPARING SORBITOL LIQUID AND LIQUID POLYOL BY USING MALTITOL RAFFINATE**
VERFAHREN ZUM HERSTELLEN VON SORBITOLFLÜSSIGKEIT UND FLÜSSIGEM POLYOL DURCH VERWENDUNG VON MALTITOLRAFFINAT
PROCÉDÉ DE PRÉPARATION D'UN LIQUIDE DE SORBITOL ET DE POLYOL LIQUIDE À L'AIDE D'UN RAFFINAT DE MALTITOL

(30) Priority: 11.02.2020 CN 202010087372
(43) Date of publication of application: 06.07.2022
(73) Proprietor: Zhejiang Huakang Pharmaceutical Co., Ltd., Quzhou, Zhejiang 324302 (CN)
(72) Inventor: MAO, Baoxing, Quzhou, Zhejiang 324302 (CN); WANG, Xiuxiu, Quzhou, Zhejiang 324302 (CN); CHEN, Deshui, Quzhou, Zhejiang 324302 (CN); LUO, Jiaxing, Quzhou, Zhejiang 324302 (CN); CHENG, Xinping, Quzhou, Zhejiang 324302 (CN)
(74) Representative: Sun, Yiming
(86) International application number: PCT/CN2021/075694
(87) International publication number: WO 2021/160052

(56) References cited:
- EP-A1- 1 176 131
- CN-A- 104 450 799
- CN-A- 104 450 799
- CN-A- 105 561 894
- CN-A- 111 206 056
- US-A- 4 322 569
- US-A- 5 932 015
- An Kehong: "Biotransformation of Inulin and Cassava Starch into High Titer Sorbitol and Gluconic Acid", Chinese Master's Theses Full-text Database, Tianjin Polytechnic University, CN, 15 December 2013 (2013-12-15), XP055835265, CN ISSN: 1674-0246

## Description

### TECHNICAL FIELD

The present invention relates to the field of recycling technologies of maltitol raffinates, and in particular to a method of preparing a sorbitol liquid and a liquid polyol using a maltitol raffinate.

### BACKGROUND

Maltitol is a disaccharide alcohol prepared by performing hydrogenation reduction for maltose. With advent and development of chromatography technology, a method of preparing a crystal maltitol by use of chromatographic separation has drawn broad attentions of people.

A given amount of chromatographic raffinate may be generated as a byproduct when a high purity maltitol component is prepared by use of chromatographic separation. The chromatographic raffinate byproduct has no high added value at present. The main components of the chromatographic raffinate are oligosaccharide alcohol (20-30%), sorbitol (20-30%), and maltitol (30-40%). The main process of maltitol is as follows:

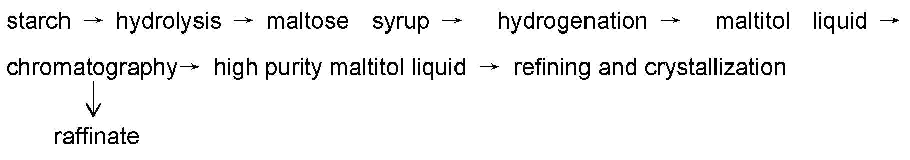

In order to increase the added value of the raffinate, a patent with a publication number CN104177229B provides a process of preparing a solid oligosaccharide alcohol and a solid sorbitol, in which the solid oligosaccharide alcohol and the solid sorbitol are prepared using a residue liquid of maltitol chromatographic separation such that an added-value use of the residue liquid of maltitol chromatographic separation can be realized. However, multiple components are obtained by performing separation and purification for the residue liquid based on chromatographic separation technology in this method, leading to increased complexity and increased difficulty of operation of a system. Further, one set of chromatography equipment is required, increasing the investment.

### SUMMARY

In order to solve the above technical problems, the present invention provides a method of preparing a sorbitol liquid and a liquid polyol using a maltitol raffinate, so as to effectively utilize the maltitol raffinate, and increase its added value. This method is simple, practical and low in costs.

The present invention is achieved by providing a method of preparing a sorbitol liquid and a liquid polyol using a maltitol raffinate. The method includes the following steps:
at step 1, obtaining a saccharification liquid by adding glucoamylase to the maltitol raffinate for saccharification;
at step 2, obtaining a fermentation liquid by adding a dry yeast to the above saccharification liquid for fermentation; and
at step 3, obtaining the sorbitol liquid and the liquid polyol by performing decolorization filtration and membrane separation for the above fermentation liquid.

Furthermore, the method includes the following steps:
at step 1, adding glucoamylase to the maltitol raffinate and performing saccharification at a temperature of 55-65 °C for 22-25 hours to obtain a saccharification liquid;
at step 2, adding a dry yeast to the above saccharification liquid and performing fermentation at a temperature of 30-40°C for 60-65 hours to obtain a fermentation liquid; and
at step 3, obtaining a diluted liquid and a concentrated liquid by performing decolorizaiton filtration and membrane separation for the above fermentation liquid, wherein the diluted liquid is the sorbitol liquid, and the concentrated liquid is the liquid polyol.

Compared with prior art, the method of preparing a sorbitol liquid and a liquid polyol using a maltitol raffinate according to the present invention has the following advantages.
1) The components of the maltitol raffinate are fully utilized, increasing the added value of the product.
2) Before membrane separation, a given amount of glucoamylase is added for saccharification and then a given amount of yeast is added for fermentation, such that the contents of the total sugar and the reducing sugar in subsequent product are reduced, thus increasing the purities of the sorbitol liquid and the liquid polyol.

### DETAILED DESCRIPTION OF THE EMBODIMENTS

In order to make the technical problems, the technical solutions and the beneficial effects of the present invention clearer and more understandable, the present invention will be further described in combination with specific embodiments.

A preferred embodiment of the method of preparing a sorbitol liquid and a liquid polyol using a maltitol raffinate according to the present invention includes the following steps.

At step 1, glucoamylase is added to the maltitol raffinate for saccharification to obtain a saccharification liquid.

At step 2, a fermentation liquid is obtained by adding a dry yeast to the above saccharification liquid for fermentation.

At step 3, the sorbitol liquid and the liquid polyol are obtained by performing decolorization filtration and membrane separation for the above fermentation liquid.

Specifically, the preferred embodiment of the method of preparing a sorbitol liquid and a liquid polyol using a maltitol raffinate according to the present invention includes the following steps.

At step 1, glucoamylase with a mass being equal to 0.04-0.16% of a mass of the maltitol raffinate is added to the maltitol raffinate and saccharification is performed at a temperature of 55-65°C for 22-25 hours to obtain a saccharification liquid, where oligosaccharide alcohol therein is hydrolyzed and a content of oligosaccharide alcohol measured in the saccharification liquid is reduced by 40-45%.

At step 2, a dry yeast with a mass being equal to 0.4-0.6% of the mass of the maltitol raffinate is added to the above saccharification liquid, and fermentation is performed at a temperature of 30-40°C for 60-65 hours to obtain a fermentation liquid, wherein a content of a reducing sugar measured in the fermentation liquid is reduced to below 0.2%.

At step 3, a diluted liquid and a concentrated liquid are obtained by performing decolorizaiton filtration and membrane separation for the above fermentation liquid, wherein the diluted liquid is the sorbitol liquid, and the concentrated liquid is the liquid polyol.

The components of the diluted liquid and the concentrated liquid are measured as follows:

| | Maltitol | Sorbitol | Oligosaccharide alcohol | Reducing surgar |
|---|---|---|---|---|
| Diluted liquid | 9-14% | 70-80% | 5-10% | 0.15-0.20% |
| Concentrated liquid | 40-50% | 13-18% | 35-40% | 0.07-0.15% |

The diluted liquid conforms to the state standard regarding sorbitol liquid in terms of components and can be directly used as a sorbitol liquid in a product. The concentrated liquid may be used as a multi-component liquid polyol in food processing due to a low content of reducing sugar and high contents of maltitol and oligosaccharide alcohol, so as to achieve the effects of moistening, increasing service life and increasing sweetness and the like. Because it is a sugar alcohol, it can also achieve the effect of reducing heat.

The method of preparing a sorbitol liquid and a liquid polyol using a maltitol raffinate according to the present invention will be further described in combination with the specific embodiments.

### Embodiment 1

The first embodiment of the method of preparing a sorbitol liquid and a liquid polyol using a maltitol raffinate according to the present invention includes the following steps.

At step 1, 1kg of the maltitol raffinate was taken, 0.4g of glucoamylase was added, and saccharification was performed at a constant temperature of 60°C for 24 hours to obtain a saccharification liquid, wherein a content of oligosaccharide alcohol measured in the saccharification liquid was reduced by 40%;

At step 2, 4g of a dry yeast was added to the above saccharification liquid, and fermentation was performed at a temperature of 30°C for 65 hours to obtain a fermentation liquid; wherein a content of a reducing sugar was measured to be 0.15%.

At step 3, decolorization filtration was performed to remove the yeast in the fermentation liquid and then membrane separation was performed to obtain a diluted liquid and a concentrated liquid respectively, wherein the diluted liquid was the sorbitol liquid and the concentrated liquid was the liquid polyol.

The components of the diluted liquid and the concentrated liquid were measured as follows:

| | Maltitol | Sorbitol | Oligosaccharide alcohol | Reducing surgar |
|---|---|---|---|---|
| Diluted liquid | 10% | 75% | 10% | 0.17% |
| Concentrated liquid | 45% | 13% | 35% | 0.09% |

### Embodiment 2

The second embodiment of the method of preparing a sorbitol liquid and a liquid polyol using a maltitol raffinate according to the present invention includes the following steps.

At step 1, 1kg of the maltitol raffinate was taken, 1g of glucoamylase was added, and saccharification was performed at a constant temperature of 55°C for 25 hours to obtain a saccharification liquid, wherein a content of oligosaccharide alcohol measured in the saccharification liquid was reduced by 45%.

At step 2, 5g of a dry yeast was added to the above saccharification liquid and fermentation was performed at a temperature of 40°C for 60 hours to obtain a fermentation liquid, wherein a content of a reducing sugar was measured to be 0.12%.

At step 3, decolorization filtration was performed to remove the yeast in the fermentation liquid and then membrane separation was performed to obtain a diluted liquid and a concentrated liquid respectively, wherein the diluted liquid was the sorbitol liquid and the concentrated liquid was the liquid polyol.

The components of the diluted liquid and the concentrated liquid were measured as follows:

| | Maltitol | Sorbitol | Oligosaccharide alcohol | Reducing surgar |
|---|---|---|---|---|
| Diluted liquid | 11% | 78% | 8% | 0.15% |
| Concentrated liquid | 43% | 15% | 37% | 0.1% |

### Embodiment3

The third embodiment of the method of preparing a sorbitol liquid and a liquid polyol using a maltitol raffinate according to the present invention includes the following steps.

At step 1, 1kg of the maltitol raffinate was taken, 1.6g of glucoamylase was added, and saccharification was performed at a constant temperature of 65°C for 22 hours to obtain a saccharification liquid, wherein a content of oligosaccharide alcohol measured in the saccharification liquid was reduced by 43%.

At step 2, 6g of a dry yeast was added to the above saccharification liquid and fermentation was performed at a temperature of 35°C for 63 hours to obtain a fermentation liquid, wherein a content of a reducing sugar was measured to be 0.14%.

At step 3, decolorization filtration was performed to remove the yeast in the fermentation liquid and then membrane separation was performed to obtain a diluted liquid and a concentrated liquid respectively, wherein the diluted liquid was the sorbitol liquid and the concentrated liquid was the liquid polyol.

The components of the diluted liquid and the concentrated liquid were measured as follows:

| | Maltitol | Sorbitol | Oligosaccharide alcohol | Reducing surgar |
|---|---|---|---|---|
| Diluted liquid | 13% | 77% | 8% | 0.16% |
| Concentrated liquid | 48% | 15% | 36% | 0.09% |

## Claims

1. A method of preparing a sorbitol liquid and a liquid polyol using a maltitol raffinate, comprising:
at step 1, obtaining a saccharification liquid by adding glucoamylase to the maltitol raffinate for saccharification;
at step 2, obtaining a fermentation liquid by adding a dry yeast to the above saccharification liquid for fermentation;
at step 3, obtaining the sorbitol liquid and the liquid polyol by performing decolorization filtration and membrane separation for the above fermentation liquid.

2. The method of claim 1, further comprising:
at step 1, adding glucoamylase to the maltitol raffinate and performing saccharification at a temperature of 55-65 °C for 22-25 hours to obtain a saccharification liquid;
at step 2, adding a dry yeast to the above saccharification liquid and performing fermentation at a temperature of 30-40°C for 60-65 hours to obtain a fermentation liquid;
at step 3, obtaining a diluted liquid and a concentrated liquid by performing decolorization filtration and membrane separation for the above fermentation liquid, wherein the diluted liquid is the sorbitol liquid, and the concentrated liquid is the liquid polyol.

3. The method of claim 2, wherein at step 1, the added amount of glucoamylase is 0.04-0.16% of a mass of the maltitol raffinate.

4. The method of claim 2, wherein at step 1, a content of oligosaccharide alcohol measured in the saccharification liquid is reduced by 40-45%.

5. The method of claim 2, wherein at step 2, the added amount of the dry yeast is 0.4-0.6% of the mass of the maltitol raffinate.

6. The method of claim 2, wherein at step 2, a reducing sugar measured in the fermentation liquid is reduced to below 0.2%.

7. The method of claim 2, comprising the following steps:
at step 1, taking 1kg of the maltitol raffinate, adding 0.4g of glucoamylase, performing saccharification at a constant temperature of 60°C for 24 hours to obtain a saccharification liquid, wherein a content of oligosaccharide alcohol measured in the saccharification liquid is reduced by 40%;
at step 2, adding 4g of a dry yeast to the above saccharification liquid, and performing fermentation at a temperature of 30 °C for 65 hours to obtain a fermentation liquid;
at step 3, performing decolorization filtration to remove the yeast in the fermentation liquid and then performing membrane separation to obtain a diluted liquid and a concentrated liquid respectively, wherein the diluted liquid is the sorbitol liquid and the concentrated liquid is the liquid polyol.

8. The method of claim 2, comprising the following steps:
at step 1, taking 1kg of the maltitol raffinate, adding 1g of glucoamylase, and performing saccharification at a constant temperature of 55°C for 25 hours to obtain a saccharification liquid, wherein a content of oligosaccharide alcohol measured in the saccharification liquid is reduced by 45%;
at step 2, adding 5g of a dry yeast to the above saccharification liquid and performing fermentation at a temperature of 40 °C for 60 hours to obtain a fermentation liquid;
at step 3, performing decolorization filtration to remove the yeast in the fermentation liquid and then performing membrane separation to obtain a diluted liquid and a concentrated liquid respectively, wherein the diluted liquid is the sorbitol liquid and the concentrated liquid is the liquid polyol.

9. The method of claim 2, comprising the following steps:
at step 1, taking 1kg of the maltitol raffinate, adding 1.6g of glucoamylase, and performing saccharification at a constant temperature of 65°C for 22 hours to obtain a saccharification liquid, wherein a content of oligosaccharide alcohol measured in the saccharification liquid is reduced by 43%;
at step 2, adding 6g of a dry yeast to the above saccharification liquid and performing fermentation at a temperature of 35 °C for 63 hours to obtain a fermentation liquid;
at step 3, performing decolorization filtration to remove the yeast in the fermentation liquid and then performing membrane separation to obtain a diluted liquid and a concentrated liquid respectively, wherein the diluted liquid is the sorbitol liquid and the concentrated liquid is the liquid polyol.

## Patentansprüche

1. Verfahren zur Herstellung einer Sorbitol-Flüssigkeit und eines flüssigen Polyols unter Verwendung eines Maltitolraffinats, umfassend:
in Schritt 1, das Gewinnen einer Verzuckerungsflüssigkeit durch das Zugeben von Glucoamylase zu dem Maltitolraffinat zur Verzuckerung;
in Schritt 2, das Gewinnen einer Fermentationsflüssigkeit durch das Zugeben einer Trockenhefe zu der obigen Verzuckerungsflüssigkeit zur Fermentierung;
in Schritt 3, das Gewinnen der Sorbitolflüssigkeit und des flüssigen Polyols durch das Durchführen einer Entfärbungsfiltration und Membrantrennung für die obige Fermentationsflüssigkeit.

2. Verfahren nach Anspruch 1, ferner umfassend:
in Schritt 1, das Zugeben von Glucoamylase zu dem Maltitolraffinat und das Durchführen der Verzuckerung bei einer Temperatur von 55-65 °C für 22-25 Stunden, um eine Verzuckerungsflüssigkeit zu erhalten;
in Schritt 2, das Zugeben einer Trockenhefe zu der obigen Verzuckerungsflüssigkeit und das Durchführen einer Fermentation bei einer Temperatur von 30-40 °C für 60-65 Stunden, um eine Fermentationsflüssigkeit zu erhalten;
in Schritt 3, das Erhalten einer verdünnten Flüssigkeit und einer konzentrierten Flüssigkeit durch das Durchführen einer Entfärbungsfiltration und einer Membrantrennung für die obige Fermentationsflüssigkeit, wobei die verdünnte Flüssigkeit die Sorbitolflüssigkeit ist und die konzentrierte Flüssigkeit das flüssige Polyol ist.

3. Verfahren nach Anspruch 2, wobei in Schritt 1 die zugesetzte Menge an Glucoamylase 0,04-0,16 % der Masse des Maltitolraffinats beträgt.

4. Verfahren nach Anspruch 2, wobei in Schritt 1 ein Gehalt an Oligosaccharidalkohol, der in der Verzuckerungsflüssigkeit gemessen wird, um 40-45 % reduziert wird.

5. Verfahren nach Anspruch 2, wobei in Schritt 2 die zugesetzte Menge der Trockenhefe 0,4-0,6 % der Masse des Maltitolraffinats beträgt.

6. Verfahren nach Anspruch 2, wobei in Schritt 2 ein reduzierender Zucker, der in der Fermentationsflüssigkeit gemessen wird, auf unter 0,2 % reduziert wird.

7. Verfahren nach Anspruch 2, umfassend die folgenden Schritte:
in Schritt 1, das Entnehmen von 1 kg des Maltitolraffinats, das Zugeben von 0,4 g Glucoamylase, das Durchführen der Verzuckerung bei einer konstanten Temperatur von 60 °C für 24 Stunden, um eine Verzuckerungsflüssigkeit zu erhalten, wobei ein Gehalt an Oligosaccharidalkohol, der in der Verzuckerungsflüssigkeit gemessen wird, um 40 % reduziert wird;
in Schritt 2, das Zugeben von 4 g einer Trockenhefe zu der obigen Verzuckerungsflüssigkeit und das Durchführen einer Fermentation bei einer Temperatur von 30 °C für 65 Stunden, um eine Fermentationsflüssigkeit zu erhalten;
in Schritt 3, das Durchführen einer Entfärbungsfiltration, um die Hefe in der Fermentationsflüssigkeit zu entfernen, und dann das Durchführen einer Membrantrennung, um eine verdünnte Flüssigkeit bzw. eine konzentrierte Flüssigkeit zu erhalten, wobei die verdünnte Flüssigkeit die Sorbitolflüssigkeit und die konzentrierte Flüssigkeit das flüssige Polyol ist.

8. Verfahren nach Anspruch 2, umfassend die folgenden Schritte:
in Schritt 1, das Entnehmen von 1 kg Maltitolraffinat, das Zugeben von 1 g Glucoamylase und das Durchführen der Verzuckerung bei einer konstanten Temperatur von 55 °C für 25 Stunden, um eine Verzuckerungsflüssigkeit zu erhalten, wobei ein Gehalt an Oligosaccharidalkohol, der in der Verzuckerungsflüssigkeit gemessen wird, um 45 % reduziert wird;
in Schritt 2, das Zugeben von 5 g einer Trockenhefe zu der obigen Verzuckerungsflüssigkeit und das Durchführen einer Fermentation bei einer Temperatur von 40 °C für 60 Stunden, um eine Fermentationsflüssigkeit zu erhalten;
in Schritt 3, das Durchführen einer Entfärbungsfiltration, um die Hefe in der Fermentationsflüssigkeit zu entfernen, und dann das Durchführen einer Membrantrennung, um eine verdünnte Flüssigkeit bzw. eine konzentrierte Flüssigkeit zu erhalten, wobei die verdünnte Flüssigkeit die Sorbitolflüssigkeit und die konzentrierte Flüssigkeit das flüssige Polyol ist.

9. Verfahren nach Anspruch 2, umfassend die folgenden Schritte:
in Schritt 1, das Entnehmen von 1 kg Maltitolraffinat, das Zugeben von 1,6 g Glucoamylase und das Durchführen der Verzuckerung bei einer konstanten Temperatur von 65 °C für 22 Stunden, um eine Verzuckerungsflüssigkeit zu erhalten, wobei ein Gehalt an Oligosaccharidalkohol, der in der Verzuckerungsflüssigkeit gemessen wird, um 43 % reduziert wird;
in Schritt 2, das Zugeben von 6 g einer Trockenhefe zu der obigen Verzuckerungsflüssigkeit und das Durchführen einer Fermentation bei einer Temperatur von 35 °C für 63 Stunden, um eine Fermentationsflüssigkeit zu erhalten;
in Schritt 3, das Durchführen einer Entfärbungsfiltration, um die Hefe in der Fermentationsflüssigkeit zu entfernen, und dann das Durchführen einer Membrantrennung, um eine verdünnte Flüssigkeit bzw. eine konzentrierte Flüssigkeit zu erhalten, wobei die verdünnte Flüssigkeit die Sorbitolflüssigkeit und die konzentrierte Flüssigkeit das flüssige Polyol ist.

## Revendications

1. Procédé de préparation d'un liquide de sorbitol et d'un polyol liquide à l'aide d'un raffinat de maltitol comprenant :
à l'étape 1, obtention d'un liquide de saccharification par ajout de glucoamylase au raffinat de maltitol pour la saccharification ;
à l'étape 2, obtention d'un liquide de fermentation par ajout d'une levure sèche au liquide de saccharification ci-dessus pour la fermentation ;
à l'étape 3, obtention du liquide de sorbitol et du polyol liquide par réalisation d'une filtration décolorante et d'une séparation sur membrane pour le liquide de fermentation ci-dessus.

2. Procédé selon la revendication 1, comprenant en outre :
à l'étape 1, ajout de glucoamylase au raffinat de maltitol et réalisation d'une saccharification à une température de 55 à 65 °C pendant 22 à 25 heures pour obtenir un liquide de saccharification ;
à l'étape 2, ajout d'une levure sèche au liquide de saccharification ci-dessus et réalisation d'une fermentation à une température de 30 à 40 °C pendant 60 à 65 heures pour obtenir un liquide de fermentation ;
à l'étape 3, obtention d'un liquide dilué et d'un liquide concentré par réalisation d'une filtration décolorante et d'une séparation sur membrane pour le liquide de fermentation ci-dessus, dans lequel le liquide dilué est le liquide de sorbitol, et le liquide concentré est le polyol liquide.

3. Procédé selon la revendication 2, dans lequel à l'étape 1, la quantité ajoutée de glucoamylase est de 0,04 à 0,16 % d'une masse du raffinat de maltitol.

4. Procédé selon la revendication 2, dans lequel à l'étape 1, une teneur en alcool oligosaccharidique mesurée dans le liquide de saccharification est réduite de 40 à 45 %.

5. Procédé selon la revendication 2, dans lequel à l'étape 2, la quantité ajoutée de la levure sèche est de 0,4 à 0,6 % de la masse du raffinat de maltitol.

6. Procédé selon la revendication 2, dans lequel à l'étape 2, un sucre réducteur mesuré dans le liquide de fermentation est réduit au-dessous de 0,2 %.

7. Procédé selon la revendication 2, comprenant les étapes suivantes :
à l'étape 1, prise de 1 kg du raffinat de maltitol, ajout de 0,4 g de glucoamylase, réalisation d'une saccharification à une température constante de 60 °C pendant 24 heures pour obtenir un liquide de saccharification, dans lequel une teneur en alcool oligosaccharidique mesurée dans le liquide de saccharification est réduite de 40 % ;
à l'étape 2, ajout de 4 g d'une levure sèche au liquide de saccharification ci-dessus, et réalisation d'une fermentation à une température de 30 °C pendant 65 heures pour obtenir un liquide de fermentation ;
à l'étape 3, réalisation d'une filtration décolorante pour retirer la levure du liquide de fermentation puis réalisation d'une séparation sur membrane pour obtenir un liquide dilué et un liquide concentré respectivement, dans lequel le liquide dilué est le liquide de sorbitol et le liquide de concentré est le polyol liquide.

8. Procédé selon la revendication 2, comprenant les étapes suivantes :
à l'étape 1, prise de 1 kg du raffinat de maltitol, ajout de 1 g de glucoamylase, et réalisation d'une saccharification à une température constante de 55 °C pendant 25 heures pour obtenir un liquide de saccharification, dans lequel une teneur en alcool oligosaccharidique mesurée dans le liquide de saccharification est réduite de 45 % ;
à l'étape 2, ajout de 5 g d'une levure sèche au liquide de saccharification ci-dessus et réalisation d'une fermentation à une température de 40 °C pendant 60 heures pour obtenir un liquide de fermentation ;
à l'étape 3, réalisation d'une filtration décolorante pour retirer la levure du liquide de fermentation puis réalisation d'une séparation sur membrane pour obtenir un liquide dilué et un liquide concentré respectivement, dans lequel le liquide dilué est le liquide de sorbitol et le liquide de concentré est le polyol liquide.

9. Procédé selon la revendication 2, comprenant les étapes suivantes :
à l'étape 1, prise de 1 kg du raffinat de maltitol, ajout de 1,6 g de glucoamylase, et réalisation d'une saccharification à une température constante de 65 °C pendant 22 heures pour obtenir un liquide de saccharification, dans lequel une teneur en alcool oligosaccharidique mesurée dans le liquide de saccharification est réduite de 43%;
à l'étape 2, ajout de 6 g d'une levure sèche au liquide de saccharification ci-dessus et réalisation d'une fermentation à une température de 35 °C pendant 63 heures pour obtenir un liquide de fermentation ;
à l'étape 3, réalisation d'une filtration décolorante pour retirer la levure du liquide de fermentation puis réalisation d'une séparation sur membrane pour obtenir un liquide dilué et un liquide concentré respectivement, dans lequel le liquide dilué est le liquide de sorbitol et le liquide de concentré est le polyol liquide.
